# FASCICULE DE BREVET EUROPEEN

(11) **EP 0 716 850 B1**
(45) Date de publication et mention de la délivrance du brevet: **20.08.1997**
(21) Numéro de dépôt: 95402678.7
(22) Date de dépôt: 28.11.1995
(51) Int. Cl.: A61K 7/48, A61K 31/135

(54) **Utilisation d'un dérivé de l'éthylène diamine dans une composition cosmétique ou dermatologique et composition contenant notamment un produit à effet secondaire irritant**
Verwendung eines Ethylendiaminderivats in einer kosmetischen und/oder dermatologischen Zusammensetzung und Zusammensetzung, die insbesondere ein Produkt mit reizender Nebenwirkung enthaelt
Use of an ethylene diamine derivative in a cosmetic or dermatological composition and in compositions containing materials with irritant side effects

(30) Priorité: 19.12.1994 FR 9415250
(43) Date de publication de la demande: 19.06.1996
(73) Titulaire: L'OREAL, 75008 Paris (FR)
(72) Inventeur: Galey, Jean-Baptiste, F-93600 Aulnay-sous-Bois (FR); De Laccharriere, Olivier, F-75015 Paris (FR); Breton, Lionel, F-78000 Versailles (FR); DUMATS Jacqueline, F-93420 Villepinte (FR)
(74) Mandataire: Lhoste, Catherine

(56) Documents cités:
- EP-A- 0 375 668
- EP-A- 0 522 808
- EP-A- 0 653 208
- WO-A-93/10073
- WO-A-94/11338

## Description

La présente invention concerne l'utilisation d'un dérivé de l'éthylène diamine comme antagoniste de substance P et/ou comme analgésique local dans ou pour la préparation d'une composition cosmétique, pharmaceutique ou dermatologique, destinée au traitement des peaux sensibles, ainsi qu'une composition cosmétique, pharmaceutique ou dermatologique contenant ce dérivé en vue de diminuer voire éliminer les effets irritants de certains produits comme des actifs utilisés dans les domaines ci-dessus.

Il est connu que certaines peaux sont plus sensibles que d'autres.Toutefois les symptômes des peaux sensibles étaient jusqu'à présent mal caractérisés et le problème de ces peaux était, de ce fait, mal défini ; personne ne connaissait exactement le processus mis en cause dans la sensibilité de la peau. Certains pensaient qu'une peau sensible était une peau qui réagissait aux produits cosmétiques, d'autres qu'il s'agissait d'une peau qui réagissait à plusieurs facteurs extérieurs, pas forcément liés aux produits cosmétiques.

Certains tests ont été essayés pour tenter de cerner les peaux sensibles, par exemple des tests à l'acide lactique et au DMSO qui sont connus pour être des substances irritantes : voir par exemple l'article de K. Lammintausta et al., Dermatoses, 1988, 36, pages 45-49; et l'article de T. Agner et J. Serup, Clinical and Experimental Dermatology, 1989, 14, pages 214-217. Mais ces tests ne permettaient pas de caractériser les peaux sensibles.

Par ailleurs, on assimilait les peaux sensibles à des peaux allergiques.

Du fait de la méconnaissance des caractéristiques des peaux sensibles, il était jusqu'à présent très difficile voire impossible de les traiter. En fait, on les traitait indirectement, par exemple en limitant dans les compositions cosmétiques ou dermatologiques l'emploi de produits à caractère irritant tels que les tensioactifs, les conservateurs ou les parfums ainsi que l'emploi de certains actifs cosmétiques ou dermatologiques.

Après de nombreux tests cliniques, la demanderesse a pu déterminer les symptômes liés aux peaux sensibles. Ces symptômes sont en particulier des signes subjectifs, qui sont essentiellement des sensations dysesthésiques. On entend par sensations dysesthésiques des sensations plus ou moins douloureuses ressenties dans une zone cutanée comme les picotements, fourmillements, démangeaisons ou prurits, brûlures, échauffements, inconforts, tiraillements, etc.

La demanderesse a pu montrer en outre qu'une peau sensible n'était pas une peau allergique. En effet, une peau allergique est une peau qui réagit à un agent extérieur, un allergène, qui déclenche une réaction d'allergie. Il s'agit d'un processus immunologique qui ne se produit que lorsqu'un allergène est présent et qui ne touche que les sujets sensibilisés. La caractéristique essentielle de la peau sensible est selon la demanderesse, au contraire, un mécanisme de réponse à des facteurs extérieurs, qui peut concerner tout individu, même si les individus dits à peau sensible y réagissent plus vite que les autres. Ce mécanisme n'est pas immunologique, il est aspécifique.

La demanderesse a maintenant trouvé que les peaux sensibles pouvaient être scindées en deux grandes formes cliniques, les peaux irritables et/ou réactives, et les peaux intolérantes.

Une peau irritable et/ou réactive est une peau qui réagit par un prurit, c'est-à-dire par des démangeaisons, ou par des picotements, à différents facteurs tels que l'environnement, les émotions, les aliments, le vent, les frottements, le rasoir, le savon, les tensioactifs, l'eau dure à forte concentration de calcaire, les variations de température ou la laine. En général, ces signes sont associés à une peau sèche avec ou sans dartres, ou à une peau qui présente un érythème.

Une peau intolérante est une peau qui réagit par des sensations d'échauffement, de tiraillements, de fourmillements et/ou de rougeurs, à différents facteurs tels que l'environnement, les émotions, les aliments et certains produits cosmétiques. En général, ces signes sont associés à une peau hyperséborrhéique ou acnéique avec ou sans dartres, et à un érythème.

Les cuirs chevelus "sensibles" ont une sémiologie clinique plus univoque : les sensations de prurit et/ou de picotements et/ou d'échauffements sont essentiellement déclenchés par des facteurs locaux tels que frottements, savon, tensioactifs, eau dure à forte concentration de calcaire, shampooings ou lotions. Ces sensations sont aussi parfois déclenchées par des facteurs tels que l'environnement, les émotions et/ou les aliments. Un érythème et une hyperséborrhée du cuir chevelu ainsi qu'un état pelliculaire sont fréquemment associés aux signes précédents.

Par ailleurs, dans certaines régions anatomiques comme les grands plis (régions inguinales, génitale, axillaires, poplitées, anale, sous-mammaires, plis du coude) et les pieds, la peau sensible se traduit par des sensations prurigineuses et/ou des sensations dysesthésiques (échauffement, picotements) liées en particulier à la sueur, aux frottements, à la laine, aux tensioactifs, à certaines préparations cosmétiques, à l'eau dure à forte concentration en calcaire et/ou aux variations de température.

Pour déterminer si une peau est sensible ou non, la demanderesse a également mis au point un test. En effet, après avoir effectué un grand nombre de tests dans le but de définir une peau sensible, elle a trouvé de manière surprenante qu'il existait un lien entre les personnes à peau sensible et celles qui réagissaient à une application topique de capsaïcine.

Le test à la capsaïcine consiste à appliquer sur environ 4 cm² de peau 0,05 ml d'une crème contenant 0,075 % de capsaïcine et à noter l'apparition de signes subjectifs provoqués par cette application, tels que picotements, brûlures et démangeaisons. Chez les sujets à peaux sensibles, ces signes apparaissent entre 3 et 20 minutes après l'application et sont suivis de l'apparition d'un érythème qui débute à la périphérie de la zone d'application.

Jusqu'à présent, la capsaïcine était utilisée comme médicament, en particulier pour traiter les douleurs du zona. La capsaïcine provoque un relargage des neuropeptides, et en particulier des tachykinines qui proviennent de terminaisons nerveuses de l'épiderme et du derme. La demanderesse a constaté que le schéma physiopathologique commun à tous les états des peaux sensibles était lié à une grande aptitude à libérer des tachykinines et plus particulièrement de la substance P dans la peau. Les manifestations dysesthésiques qui sont provoquées par leur libération sont dites "neurogènes".

Cette substance P est un élément chimique polypeptidique élaboré et libéré par une terminaison nerveuse. Elle fait partie de la famille des tachykinines. La substance P intervient notamment dans la transmission de la douleur et dans des maladies du système nerveux central tels que l'anxiété, la schizophrénie, dans des maladies respiratoires et inflammatoires, dans des maladies gastrointestinales, dans des maladies rhumatismales et dans certaines maladies dermatologiques telles que l'eczéma.

La demanderesse a maintenant découvert que la caractéristique essentielle des peaux sensibles était liée à la libération de substance P et donc que l'utilisation d'antagonistes de substance P pouvait permettre d'obtenir un effet préventif et/ou curatif des peaux sensibles.

Pour traiter les peaux sensibles, la demanderesse a donc envisagé d'utiliser des antagonistes de substance P. Elle a en effet constaté de manière surprenante que l'incorporation d'un antagoniste de substance P dans une composition cosmétique, pharmaceutique ou dermatologique permettait d'éviter l'irritation et/ou les sensations dysesthésiques et/ou les prurits de la peau.

Il est connu du document WO 93/10073 des dérivés de l'éthylène diamine utilisables comme antagonistes de substances P dans des compositions pharmaceutiques pour le traitement et la prévention des désordres du système nerveux central. Ces composés présentent l'inconvénient d'être souvent difficiles à fabriquer ou formuler dans une composition cosmétique, pharmaceutique ou dermatologique. En outre ce document ne décrit ni suggère l'utilisation de certains de ces dérivés dans des compositions à application topique.

On connait, par le document EP-A-653208, certains dérivés de l'éthylène diamine utilisables dans le traitement et la prévention des brûlures solaires. Toutefois, ce document ne décrit ni ne suggère l'utilisation de ces dérivés dans des compositions à application topique pour le traitement des peaux sensibles.

La présente invention a donc pour objet l'utilisation d'au moins un composé de formule (I) suivante ou de l'un de ses sels cosmétiquement, dermatologiquement ou pharmaceutiquement acceptable, comme antagoniste de substance P dans ou pour la préparation d'une composition cosmétique, dermatologique ou pharmaceutique pour traiter les symptômes liés aux peaux sensibles : 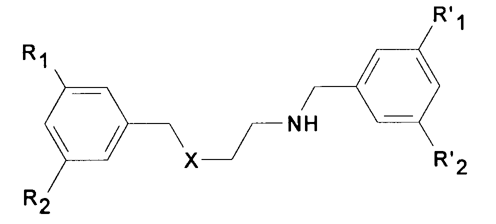
R₁, R₂ sont indépendamment l'un de l'autre H, -CH3, -OCH3, -CF3,CN, -NO2, un atome d'halogène
R'₁, R'₂ sont indépendamment l'un de l'autre -CH3, -OCH3, -CF3, -CN, -NO2, un atome d'halogène
X représente O ou NH

De préférence X représente NH. En particulier, le composé de formule (I) est choisi parmi la N,N'-bis-di-(3,5-diméthylbenzyle) éthylène diamine et la N,N'-bis-di-(3,5-diméthoxylbenzyle) éthylène diamine.

Les sels du composé de formule (I) sont notamment les chlorhydrates,les sulfates, les phosphates, les acétates, les carbonates.

La présente invention a encore pour objet l'utilisation d'au moins un composé de formule (I) ci-dessus comme analgésique local dans ou pour la préparation d'une composition cosmétique, pharmaceutique ou dermatologique pour le traitement des peaux sensibles.

La présente invention a encore pour objet l'utilisation d'au moins un composé de formule (I) ci-dessus dans ou pour la préparation d'une composition cosmétique, pharmaceutique ou dermatologique pour prévenir et/ou lutter contre les irritations cutanées et/ou les dartres et/ou les érythèmes d'origine neurogène et/ou les sensations d'échauffement et/ou de dysesthésie et/ou les prurits de la peau et/ou les muqueuses.

La composition de l'invention contient un milieu cosmétiquement, pharmaceutiquement ou dermatologiquement acceptable, c'est-à-dire un milieu compatible avec les tissus, les muqueuses, la peau, les ongles et les cheveux. Ainsi, la composition contenant un ou plusieurs composés de formule (I) peut être injectée, ingérée, ou appliquée sur le visage, le cou, les cheveux et les ongles, les grans plis ou toute autre zone cutanée du corps, les muqueuses (buccale, jugale, gingivale, génitale, conjonctive).

Pour qu'une substance soit reconnue comme un antagoniste de substance P, elle doit répondre aux caractéristiques suivantes :
- avoir une affinité sélective pour les récepteurs NK1 des tachykinines,
- avoir une activité pharmacologique antagoniste de la substance P, c'est-à-dire induire une réponse pharmacologique cohérente dans au moins l'un des deux tests suivants :
   - la substance antagoniste doit diminuer l'extravasation du plasma au travers de la paroi vasculaire induite par la capsaïcine ou par une stimulation nerveuse antidromique, ou bien
   - la substance antagoniste doit provoquer une inhibition de la contraction des muscles lisses induites par l'administration de substance P.

Les composés de formule (I) ou leurs sels satisfont bien à ces conditions.

Personne jusqu'à ce jour n'avait établi un lien entre la substance P et la peau sensible. Les signes cliniques de la peau sensible sont essentiellement subjectifs : picotements, fourmillements, prurits, tiraillements, échauffements, et ils s'associent parfois à des érythèmes. Ces signes sont dûs à des facteurs extérieurs aspécifiques. Les symptômes apparaissent essentiellement localisés au visage, au cou et au cuir chevelu, mais peuvent apparaître aussi sur tout le corps.

De façon avantageuse, les composés de formule (I) ou leurs sels sont associés à des produits à effet secondaire irritant, utilisés couramment dans le domaine cosmétique, pharmaceutique ou dermatologique et plus spécialement à des actifs cosmétiques, pharmaceutiques ou dermatologiques. La présence d'un antagoniste de substance P de formule (I) notamment salifié dans une composition cosmétique, pharmaceutique ou dermatologique contenant un produit ayant un effet irritant permet d'atténuer fortement, voire de supprimer cet effet irritant. Il permet notamment d'augmenter la quantité d'actif à effet irritant par rapport à la quantité d'actif normalement utilisée, en vue d'une efficacité améliorée.

Aussi, l'invention a encore pour objet une composition cosmétique, pharmaceutique ou dermatologique contenant dans un milieu cosmétiquement ou pharmaceutiquement acceptable au moins un produit à effet secondaire irritant, caractérisée en ce qu'elle contient en outre au moins un composé de formule (I) ci-dessus éventuellement salifié.

La présente invention a aussi pour objet l'utilisation d'au moins un composé de formule (I) ci-dessus ou de l'un de ses sels cosmétiquement, pharmaceutiquement ou dermatologiquement acceptable dans ou pour la préparation d'une composition cosmétique, pharmaceutique ou dermatologique contenant au moins un produit à effet secondaire irritant, pour diminuer et/ou éliminer cet effet irritant.

En particulier, les produits à effet secondaire irritant sont choisis parmi les tensioactifs (ioniques ou non-ioniques), les conservateurs, les solvants organiques ou les actifs comme les α-hydroxy-acides (acide citrique, malique, glycolique, tartrique, mandélique, lactique), les β-hydroxy-acides (l'acide salicylique et ses dérivés), les α-céto-acides, les β-céto-acides, les rétinoïdes (rétinol, rétinal, acide rétinoïque), les anthralines (dioxyanthranol), les anthranoïdes, les peroxydes (notamment de benzoyle), le minoxidil, les sels de lithium, les antimétabolites, la vitamine D et ses dérivés, les teintures ou colorants capillaires (paraphénylènediamine et ses dérivés, les aminophénols), les solutions alcooliques parfumantes (parfums, eaux de toilette, après rasage, déodorants), les agents antitranspirants (certains sels d'aluminium), les actifs dépilatoires ou de permanentes (thiols), les actifs dépigmentants (hydroquinone).

L'emploi d'antagoniste de substance P permet notamment de multiplier de 2 à 10 fois la quantité d'actif à effet secondaire irritant par rapport à l'état de la technique, sans ressentir tous les inconforts mentionnés ci-dessus. Ainsi, on peut utiliser les hydroxyacides jusqu'à 50 % du poids de la composition ou les rétinoïdes jusqu'à 5 %, sans aucune gêne.

Dans les compositions de l'invention, le composé de formule (I) ou un de ses sels peut être utilisé comme antagoniste de substance P et/ou comme analgésique local de préférence en une quantité allant de 0,000001 à 5 % en poids par rapport au poids total de la composition, et en particulier en une quantité allant de 0,0001 à 0,1 % en poids par rapport au poids total de la composition.

Les compositions de l'invention peuvent se présenter sous toutes les formes galéniques normalement utilisées selon que la composition doit être ingérée, injectée ou appliquée sur la peau ou les muqueuses.

Pour une application topique, la composition peut avoir la forme notamment de solution aqueuse ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, de microémulsion, ou de dispersions vésiculaires de type ionique et/ou non ionique. Ces compositions sont préparées selon les méthodes usuelles.

Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol contenant également un agent propulseur sous pression.

Pour l'injection, la composition peut se présenter sous forme de lotion aqueuse, huileuse ou sous forme de sérum. Pour les yeux, elle peut se présenter sous forme de gouttes et pour l'ingestion, elle peut se présenter sous forme de capsules, de granulés de sirops ou de comprimés.

Les quantités des différents constituants des compositions selon l'invention sont celles classiquement utilisées dans les domaines considérés.

Ces compositions constituent notamment des crèmes de nettoyage, de protection, de traitement ou de soin pour le visage, pour les mains, pour les pieds, pour les grands plis anatomiques ou pour le corps, (par exemple crèmes de jour, crèmes de nuit, crèmes démaquillantes, crèmes de fond de teint, crèmes anti-solaires), des fonds de teint fluides, des laits de démaquillage, des laits corporels de protection ou de soin, des laits anti-solaires, des lotions, gels ou mousses pour le soin de la peau, comme des lotions de nettoyage, des lotions anti-solaires, des lotions de bronzage artificiel, des compositions pour le bain, des compositions désodorisantes contenant un agent bactéricide, des gels ou lotions après-rasage, des crèmes épilatoires, des compositions contre les piqûres d'insectes, des compositions anti-douleur, des compositions pour traiter certaines maladies de la peau comme l'eczéma, la rosasée, le psoriasis, les lichens, les prurits sévères.

Les compositions selon l'invention peuvent également consister en des préparations solides constituant des savons ou des pains de nettoyage.

Les compositions peuvent aussi être conditionnées sous forme de composition pour aérosol contenant également un agent propulseur sous pression.

Le composé de l'invention peut aussi être incorporé dans diverses compositions pour soins capillaires, et notamment des shampooings, des lotions de mise en plis, des lotions traitantes, des crèmes ou des gels coiffants, des compositions de teintures (notamment teintures d'oxydation) éventuellement sous forme de shampooings colorants, des lotions restructurantes pour les cheveux, des compositions de permanente (notamment des compositions pour le premier temps d'une permanente), des lotions ou des gels antichute, des shampooings antiparasitiares, etc.

Les compositions de l'invention peuvent aussi être à usage bucco-dentaire, par exemple une pâte dentifrice. Dans ce cas, les compositions peuvent contenir des adjuvants et additifs usuels pour les compositions à usage buccal et notamment des agents tensioactifs, des agents épaississants, des agents humectants, des agents de polissage tels que la silice, divers ingrédients actifs comme les fluorures, en particulier le fluorure de sodium, et éventuellement des agents édulcorants comme le saccharinate de sodium.

Lorsque la composition de l'invention est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition de l'invention est une solution ou un gel huileux, la phase grasse peut représenter plus de 90 % du poids total de la composition.

De façon connue, la composition cosmétique de l'invention peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauda ou paraffine. On peut ajouter à ces huiles des alcools gras et des acides gras (acide stéarique).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.

Comme gélifiants hydrophiles, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe,.éthylcellulose, polyéthylène.

La composition de l'invention peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

On peut entre autres associer le composé de formule (I) éventuellement salifié à d'autres agents actifs destinés notamment à la prévention et/ou au traitement des affections cutanées. Parmi ces agents actifs, on peut citer à titre d'exemple :
- les agents modulant la différenciation et/ou la prolifération et/ou la pigmentation cutanée tels que l'acide rétinoïque et ses isomères, le rétinol et ses esters, la vitamine D et ses dérivés, les estrogènes tels que l'estradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine ;
- les agents antiviraux tels que l'acyclovir;
- les agents kératolytiques tels que les acides alpha- et bêta-hydroxy-carboxyliques ou bêta-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'alpha-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle.

La présente invention a en outre pour objet un procédé de traitement cosmétique, caractérisé par le fait que l'on applique sur la peau, sur les cheveux, et/ou sur les muqueuses, une composition telle que décrite ci-dessus contenant au moins un composé de formule (I) ou un de ses sels cosmétiquement acceptables dans un milieu cosmétiquement acceptable.

Le procédé de traitement cosmétique de l'invention peut être mis en oeuvre notamment en appliquant les compositions hygiéniques ou cosmétiques telles que définies ci-dessus, selon la technique d'utilisation habituelle de ces compositions. Par exemple : application de crèmes, de gels, de sérums, de lotions, de laits de démaquillage ou de compositions anti-solaires sur la peau ou sur les cheveux secs, application d'une lotion pour cheveux sur cheveux mouillés, de shampooings, ou encore application de dentifrice sur les gencives.

Les composés de l'invention peuvent être préparés comme décrit dans le document WO 94/11338 déposé au nom de la demanderesse. Dans cette demande les composés de formule (I) sont présentés comme des intermédiaires de synthèse. Avantageusement ces composés sont réalisés en seulement deux étapes. Le schéma de synthèse général est donné ci-après :

### molécules symétriques :

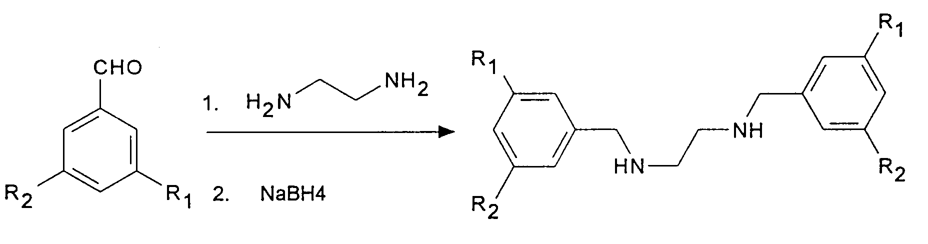

### molécules non symétriques

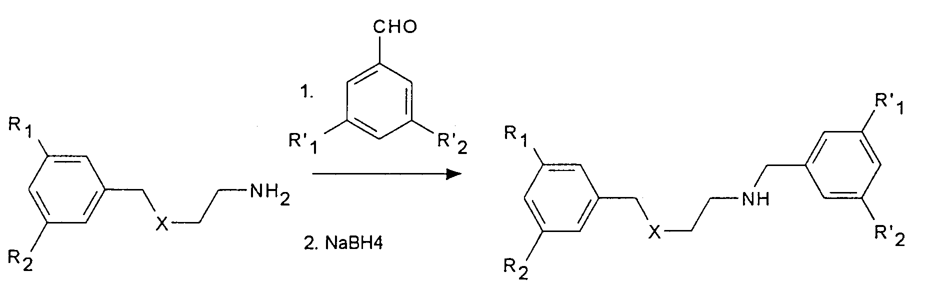

Les exemples et compositions suivants illustrent l'invention. Dans les compositions, les proportions indiquées sont des pourcentages en poids.

### Exemple 1 : Synthèse de la N,N'-bis-di-(3,5-diméthylbenzyle) éthylène diamine Ce composé correspond à R₁, R₂, R'₁, R'₂ = -CH₃ et X = NH.

### Première étape

Dans un tricol de 100 ml, on solubilise à température ambiante 5,0 g de 3,5-diméthyl benzaldéhyde dans 50 ml de méthanol. On chauffe à 50°C et on ajoute 1,25 ml d'éthylène diamine. On laisse alors revenir à température ambiante. Le milieu réactionnel devient rapidement hétérogène avec précipitation d'un solide blanc. Le produit est collecté par filtration sur verre fritté, lavé abondamment par du méthanol froid et ensuite séché sous vide au dessicateur pour obtenir 4,6 g de diimine (Rdt=85%).

### Caractérisation

RMN ¹H: δ (ppm): 2.41 (s, 12H); 3.97 (s, 4H); 7.20 (s, 2H); 7.44 (s, 4H); 8.38 (s, 2H).

### Deuxième étape

Dans un tricol de 250 ml on met en suspension à température ambiante 5,45 g de la diimine dans 50 ml d'éthanol absolu. On ajoute alors 1,10 g de borohydrure de sodium en pastilles et on agite à température ambiante pendant 15h. On concentre alors le milieu sous pression réduite puis on l'hydrolyse avec environ 50 ml d'une solution aqueuse d'HCl 6N jusqu'à pH<1. Un précipité apparait rapidement. On refroidit alors à +5°C pendant 1h environ sous faible agitation. Le précipité est filtré sur verre fritté et lavé par 50 ml d'eau glacée avant d'être séché sous vide au dessicateur. Le solide est alors remis en suspension dans 200 ml d'ether éthylique et agité pendant 2 h à température ambiante. On filtre le solide et on récupère 4,0 g de dichlorhydrate de N'N'-bis-di-(3,5-diméthylenzyl) éthylène diamine (Rdt=58%).

### Caractérisation

- RMN ¹H (d6 DMSO): δ (ppm): 2.13 (s, 12H); 3.04 (s, 4H); 3.86 (s, 4H); 6.87 (s, 2H); 7.01 (s, 4H); 8.69 (s, 2H).

### Exemple 2 : Synthèse de la N,N'-bis-di-(3,5-diméthoxybenzyle) éthylène diamine Ce composé correspond à R₁, R₂, R'₁, R'₂ = -OCH₃ et X = NH.

Selon le même procédé que pour l'exemple 1, on prépare le dichlorhydrate de la N,N'-bis-di-(3,5-dimethoxybenzyle) ethylène diamine.

### Caractérisation

- RMN ¹H (d6 DMSO): δ (ppm): 3.36 (s, 4H); 3.76 (s, 12H); 4.12 (s, 4H); 6.52 (dd, 2H); 6.82 (d, 4H); 9.88 (se, 4H).

### Activité pharmacologique des composés des exemples 1 et 2

a). Fixation réceptorielle sur récepteur NK1 humain selon la technique décrite par Heuillet et coll., J. Neurochem. 60:868-76 (1993):
   57% de fixation pour le composé de l'exemple 1 à 10 µM
   23% de fixation pour le composé de l'exemple 2 à 10 µM
b). Modèle d'antagonisme de la contraction de l'iléon de cobaye in vitro par la substance P (10 nM) selon la technique décrite par Dion et coll, Life Sci. 41:2269-78 (1987) et Patacchini et coll, Eur. J. Pharmacol. 215:93-8 (1992).

| concentration composé exemple 1 | 1µM | 10µM | 100µM |
|---|---|---|---|
| %inhibition de la contraction | -24% | -92% | -100% |

Ces tests indiquent clairement que les composés des exemples 1 et 2 sont bien des antagonistes de substance P.

| **Composition 1** :Lotion démaquillante pour le visage | |
|---|---|
| Composé de l'exemple 1 | 5,00 |
| Antioxydant | 0,05 |
| Isopropanol | 40,00 |
| Conservateur | 0,30 |
| Eau | qsp 100 % |

| **Composition 2** : Gel pour le soin du visage | |
|---|---|
| Composé de l'exemple 2 | 0,05 |
| Hydroxypropylcellulose (Klucel H vendu par la société Hercules) | 1,00 |
| Antioxydant | 0,05 |
| Isopropanol | 40,00 |
| Conservateur | 0,30 |
| Eau | qsp 100 % |

| **Composition 3** : Crème de soin du visage (émulsion huile dans eau) | |
|---|---|
| Chlorhydrate du composé de l'exemple 2 | 0,02 |
| Stéarate de glycérol | 2,00 |
| Polysorbate 60 (Tween 60 vendu par la société ICI) | 1,00 |
| Acide stéarique | 1,40 |
| Triéthanolamine | 0,70 |
| Carbomer | 0,40 |
| Fraction liquide du beurre de karité | 12,00 |
| Perhydrosqualène | 12,00 |
| Antioxydant | 0,05 |
| Parfum | 0,5 |
| Conservateur | 0,30 |
| Eau | qsp 100 % |

| **Composition 4** : Shampooing | |
|---|---|
| Sulfate du composé de l'exemple 1 | 0,02 |
| Hydroxypropylcellulose (Klucel H vendu par la société Hercules) | 1,00 |
| Parfum | 0,50 |
| Conservateur | 0,30 |
| Eau | qsp 100 % |

| **Composition 5** : Crème de soin antirides pour le visage (émulsion huile/eau) | |
|---|---|
| Composé de l'exemple 1 | 0,15 |
| Stéarate de glycérol | 2,00 |
| Polysorbate 60 (Tween 60 vendu par la société ICI) | 1,00 |
| Acide stéarique | 1,40 |
| Acide n-octanoyl-5-salicylique | 0,50 |
| Triéthanolamine | 0,70 |
| Carbomer | 0,40 |
| Fraction liquide du beurre de karité | 12,00 |
| Perhydrosqualène | 12,00 |
| Antioxydant | 0,05 |
| Parfum | 0,5 |
| Conservateur | 0,30 |
| Eau | qsp 100 % |

| **Composition 6** : Gel anti-douleur | |
|---|---|
| Composé de l'exemple 2 | 0,03 |
| Hydroxypropylcellulose (Klucel H vendu par la société Hercules) | 1,00 |
| Antioxydant | 0,05 |
| Chlorhydrate de lidocaïne | 2,00 |
| Isopropanol | 40,00 |
| Conservateur | 0,30 |
| Eau | qsp 100 % |

| **Composition 7** : Crème de soin de l'érythème solaire (émulsion huile-dans-eau) | |
|---|---|
| Composé de l'exemple 1 | 0,25 |
| Stéarate de glycérol | 2,00 |
| Polysorbate 60 (Tween 60 vendu par la société ICI) | 1,00 |
| Acide stéarique | 1,40 |
| Acide glycyrrhétinique | 2,00 |
| Triéthanolamine | 0,70 |
| Carbomer | 0,40 |
| Fraction liquide du beurre de karité | 12,00 |
| Huile de tournesol | 10,00 |
| Antioxydant | 0,05 |
| Parfum | 0,5 |
| Conservateur | 0,30 |
| Eau | qsp 100 % |

| **Composition 8** : Gel pour le traitement de l'acné | |
|---|---|
| Composé de l'exemple 2 | 5,00 |
| Acide tout trans rétinoïque | 0,05 |
| Hydroxypropylcellulose (Klucel H) | 1,00 |
| Antioxydant | 0,05 |
| Isopropanol | 40,00 |
| Conservateur | 0,30 |
| Eau | qsp 100 % |

| **Composition 9** : Lotion pour éliminer les cicatrices dues à l'acné | |
|---|---|
| Composé de l'exemple 1 | 5,00 |
| Acide glycolique | 50,00 |
| Hydroxypropylcellulose (Klucel H) | 0,05 |
| NaOH | qsp pH = 2,8 |
| Ethanol | qsp 100 % |
| Conservateur | 0,30 |

## Revendications

1. Composition cosmétique, pharmaceutique ou dermatologique contenant dans un milieu cosmétiquement ou pharmaceutiquement acceptable au moins un produit à effet secondaire irritant, caractérisée en ce qu'elle contient, en outre, au moins un composé de formule (I) suivante ou un de ses sels cosmétiquement, pharmaceutiquement ou dermatologiquement acceptable : 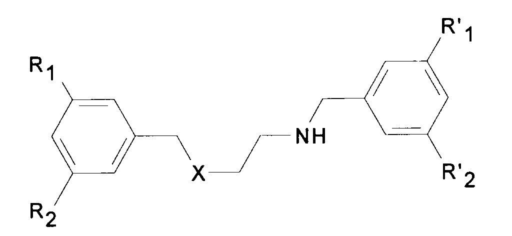
R₁, R₂ sont indépendamment l'un de l'autre H, -CH3, -OCH3, -CF3,CN, -NO2, un atome d'halogène
R'₁, R'₂ sont indépendamment l'un de l'autre -CH3, -OCH3, -CF3, -CN, -NO2, un atome d'halogène
X représente O ou NH

2. Composition selon la revendication 1, caractérisée en ce que X représente NH.

3. Composition selon la revendication 1 ou 2, caractérisée en ce que le composé de formule (I) est utilisé en une quantité allant de 0,000001 à 5 % en poids par rapport au poids total de la composition.

4. Composition selon l'une quelconque des revendications 1 à 3, caractérisée en ce que le composé de formule (I) est utilisé en une quantité allant de 0,0001 à 0,1 % en poids par rapport au poids total de la composition.

5. Composition selon l'une quelconque des revendications 1 à 4, caractérisée en ce que le produit à effet secondaire irritant est choisi parmi les α-hydroxy-acides, les β-hydroxy-acides, les α-céto-acides, les β-céto-acides, les rétinoïdes, les anthralines, les anthranoïdes, les peroxydes, le minoxidil, les sels de lithium, les antimétabolites, la vitamine D et ses dérivés, les teintures ou colorants capillaires, les agents antitranspirants, dépilatoires ou de permanente, les solutions alcooliques parfumantes, les agents dépigmentants, les tensioactifs, les solvants.

6. Composition selon l'une quelconque des revendications 1 à 5, caractérisée en ce que le produit à effet irritant secondaire est un actif cosmétique, pharmaceutique ou dermatologique.

7. Composition selon l'une quelconque des revendications 1 à 6, caractérisée en ce qu'elle contient en outre au moins un agent choisi parmi les agents antibactériens, antiparasitaires, antifongiques, antiviraux, anti-inflammatoires, antiprurigineux, anesthésiques, kératolytiques, antiradicaux libres, anti-séborrhéiques, antipelliculaires, antiacnéiques et/ou les agents modulant la différenciation et/ou la prolifération et/ou la pigmentation cutanée.

8. Composition selon l'une quelconque des revendications précédentes, caractérisée en ce que le milieu cosmétiquement ou dermatologiquement acceptable est une solution aqueuse ou hydroalcoolique, une émulsion eau-dans-huile, une émulsion huile-dans-eau, une microémulsion, un gel aqueux, un gel anhydre, un sérum, une dispersion de vésicules.

9. Utilisation comme antagoniste de substance P dans ou pour la préparation d'une composition cosmétique, pharmaceutique ou dermatologique pour traiter les symptômes liés aux peaux sensibles d'au moins un composé de formule (I) suivante ou un de ses sels cosmétiquement, pharmaceutiquement ou dermatologiquement acceptable : 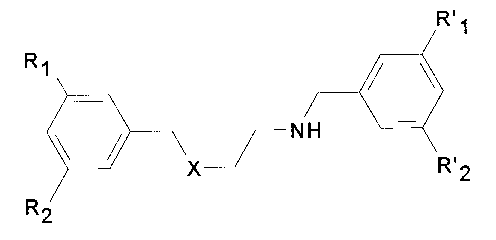
R₁, R₂ sont indépendamment l'un de l'autre H, -CH3, -OCH3, -CF3,CN, -NO2, un atome d'halogène
R'₁, R'₂ sont indépendamment l'un de l'autre -CH3, -OCH3, -CF3, -CN, -NO2, un atome d'halogène
X représente O ou NH

10. Utilisation comme analgésique local dans ou pour la préparation d'une composition cosmétique, pharmaceutique ou dermatologique pour traiter les peaux sensibles d'au moins un composé de formule (I) suivante ou un de ses sels cosmétiquement, pharmaceutiquement ou dermatologiquement acceptable : 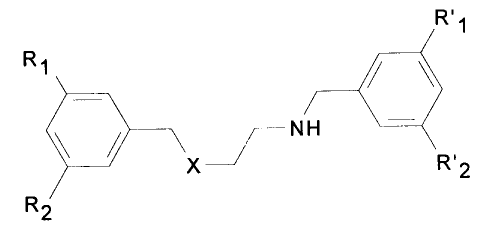
R₁, R₂ sont indépendamment l'un de l'autre H, -CH3, -OCH3, -CF3,CN, -NO2, un atome d'halogène
R'₁, R'₂ sont indépendamment l'un de l'autre -CH3, -OCH3, -CF3, -CN, -NO2, un atome d'halogène
X représente O ou NH

11. Utilisation dans ou pour la préparation d'une composition cosmétique, pharmaceutique ou dermatologique d'au moins un composé de formule (I) suivante ou un de ses sels cosmétiquement, pharmaceutiquement ou dermatologiquement acceptable, pour prévenir et/ou lutter contre les irritations cutanées et/ou les dartres et/ou les érythèmes d'origine neurogène et/ou les sensations dysesthésiques et/ou les sensations d'échauffement et/ou les prurits de la peau et/ou les muqueuses : 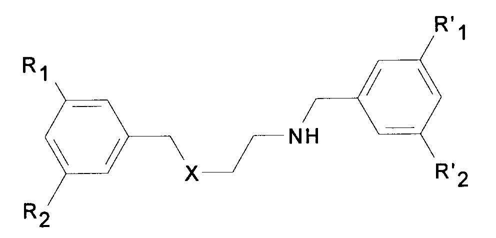
R₁, R₂ sont indépendamment l'un de l'autre H, -CH3, -OCH3, -CF3,CN, -NO2, un atome d'halogène
R'₁, R'₂ sont indépendamment l'un de l'autre -CH3, -OCH3, -CF3, -CN, -NO2, un atome d'halogène
X représente O ou NH

12. Utilisation dans ou pour la préparation d'une composition cosmétique, pharmaceutique ou dermatologique contenant au moins un produit à effet secondaire irritant, d'au moins un composé de formule (I) suivante pour diminuer et/ou éliminer cet effet irritant : 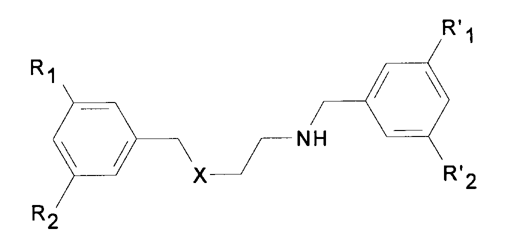
R₁, R₂ sont indépendamment l'un de l'autre H, -CH3, -OCH3, -CF3,CN, -NO2, un atome d'halogène
R'₁, R'₂ sont indépendamment l'un de l'autre -CH3, -OCH3, -CF3, -CN, -NO2, un atome d'halogène
X représente O ou NH

13. Utilisation selon l'une des revendications 9 à 12, caractérisée en ce que X représente -NH.

14. Utilisation selon l'une quelconque des revendications 9 à 13, caractérisée en ce que le composé est utilisé en une quantité allant de 0,000001 à 5 % en poids par rapport au poids total de la composition.

15. Utilisation selon l'une quelconque des revendications 9 à 14, caractérisée en ce que le composé est utilisé en une quantité allant de 0,0001 à 0,1 % en poids par rapport au poids total de la composition.

16. Utilisation selon l'une quelconque des revendications 9 à 15, caractérisée en ce que la composition contient au moins un agent choisi parmi les agents antibactériens, antiparasitaires, antifongiques, antiviraux anti-inflammatoires, antiprurigineux, anesthésiques, kératolytiques, anti-radicaux libres, anti-séborrhéiques, antipelliculaires, antiacnéiques et/ou les agents modulant la différenciation et/ou la prolifération et/ou la pigmentation cutanée.

17. Utilisation selon l'une quelconque des revendications 12 à 16, caractérisée en ce que le produit à effet secondaire irritant est choisi parmi les α-hydroxy-acides, les β-hydroxy-acides, les α-céto-acides, les β-céto-acides, les rétinoïdes, les anthralines, les anthranoïdes, les peroxydes, le minoxidil, les sels de lithium, les antimétabolites, la vitamine D et ses dérivés, les teintures ou colorants capillaires, les agents antitranspirants, dépilatoires ou de permanente, les solutions alcooliques parfumantes, les agents dépigmentants, les tensioactifs, les solvants.

18. Procédé de traitement cosmétique, caractérisé en ce que l'on applique sur la peau, sur les cheveux et/ou sur les muqueuses une composition contenant, dans un milieu cosmétiquement acceptable, au moins un composé de formule (I) suivante ou un de ses sels cosmétiquement, pharmaceutiquement ou dermatologiquement acceptable : 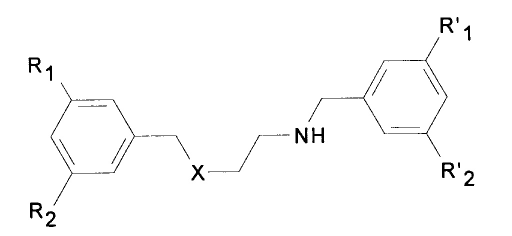
R₁, R₂ sont indépendamment l'un de l'autre H, -CH3, -OCH3, -CF3,CN, -NO2, un atome d'halogène
R'₁, R'₂ sont indépendamment l'un de l'autre -CH3, -OCH3, -CF3, -CN, -NO2, un atome d'halogène
X représente O ou NH

19. Procédé selon la revendication 18, caractérisé en ce que X représente -NH.

20. Procédé selon la revendication 18 ou 19, caractérisé en ce que le composé est utilisé en une quantité allant de 0,000001 à 5 % en poids par rapport au poids total de la composition.

21. Procédé selon l'une des revendications 18 à 20, caractérisé en ce que le composé est utilisé en une quantité allant de 0,0001 à 0,1 % en poids par rapport au poids total de la composition.

## Claims

1. Cosmetic, pharmaceutical or dermatological composition containing, in a cosmetically or pharmaceutically acceptable medium, at least one product having an irritant side effect, characterized in that it also contains at least one compound of formula (I) below or one of the cosmetically, pharmaceutically or dermatologically acceptable salts thereof: 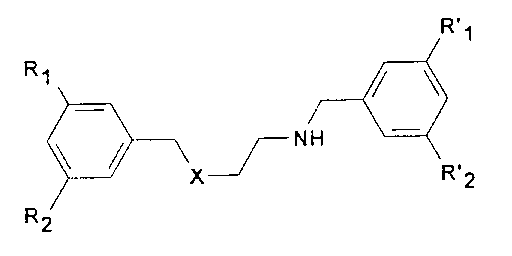
R₁ and R₂ are, independently of each other, H, -CH₃, -OCH₃, -CF₃, CN, -NO₂, a halogen atom
R'₁ and R'₂ are, independently of each other, -CH₃, -OCH₃, -CF₃, -CN, -NO₂, a halogen atom
X represents O or NH.

2. Composition according to Claim 1, characterized in that X represents NH.

3. Composition according to Claim 1 or 2, characterized in that the compound of formula (I) is used in an amount ranging from 0.000001 to 5% by weight relative to the total weight of the composition.

4. Composition according to any one of Claims 1 to 3, characterized in that the compound of formula (I) is used in an amount ranging from 0.0001 to 0.1% by weight relative to the total weight of the composition.

5. Composition according to any one of Claims 1 to 4, characterized in that the product having an irritant side effect is chosen from α-hydroxy acids, β-hydroxy acids, α-keto acids, β-keto acids, retinoids, anthralins, anthranoids, peroxides, minoxidil, lithium salts, antimetabolites, vitamin D and derivatives thereof, hair dyes or toners, antiperspirants, depilatory agents or permanent-waving agents, perfumed alcoholic solutions, depigmenting agents, surfactants and solvents.

6. Composition according to any one of Claims 1 to 5, characterized in that the product having an irritant side effect is a cosmetic, pharmaceutical or dermatological active agent.

7. Composition according to any one of Claims 1 to 6, characterized in that it also contains at least one agent chosen from antibacterial agents, antiparasitic agents, antifungal agents, antiviral agents, anti-inflammatory agents, antipruriginous agents, anaesthetics, keratolytic agents, anti-free-radical agents, antiseborrhoeic agents, antidandruff agents, antiacne agents and/or agents which modify cutaneous differentiation and/or proliferation and/or pigmentation.

8. Composition according to any one of the preceding claims, characterized in that the cosmetically or dermatologically acceptable medium is an aqueous or aqueous-alcoholic solution, a water-in-oil emulsion, an oil-in-water emulsion, a microemulsion, an aqueous gel, an anhydrous gel, a serum or a dispersion of vesicles.

9. Use as substance P antagonist in, or for the preparation of, a cosmetic, pharmaceutical or dermatological composition for treating the symptoms relating to sensitive skin-types, of at least one compound of formula (I) below or one of the cosmetically, pharmaceutically or dermatologically acceptable salts thereof: 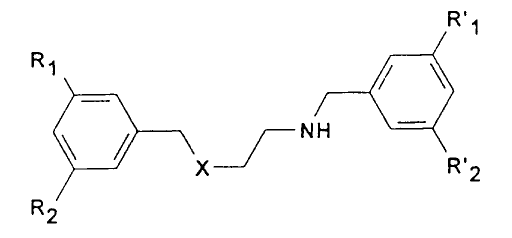
R₁ and R₂ are, independently of each other, H, -CH₃, -OCH₃, -CF₃, CN, -NO₂, a halogen atom
R'₁ and R'₂ are, independently of each other, -CH₃, -OCH₃, -CF₃, -CN, -NO₂, a halogen atom
X represents O or NH.

10. Use, as a local analgesic, in or for the preparation of a cosmetic, pharmaceutical or dermatological composition for treating sensitive skin-types, of at least one compound of formula (I) below or one of the cosmetically, pharmaceutically or dermatologically acceptable salts thereof: 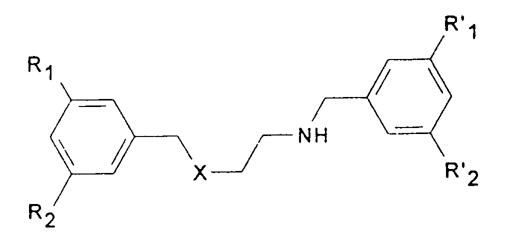
R₁ and R₂ are, independently of each other, H, -CH₃, -OCH₃, -CF₃, CN, -NO₂, a halogen atom
R'₁ and R'₂ are, independently of each other, -CH₃, -OCH₃, -CF₃, -CN, -NO₂, a halogen atom
X represents O or NH.

11. Use, in or for the preparation of a cosmetic, pharmaceutical or dermatological composition, of at least one compound of formula (I) below or one of the cosmetically, pharmaceutically or dermatologically acceptable salts thereof, for preventing and/or combating skin irritations and/or dartres and/or erythema of neurogenic origin and/or dysaesthesic sensations and/or sensations of inflammation and/or pruritus of the skin and/or the mucous membranes: 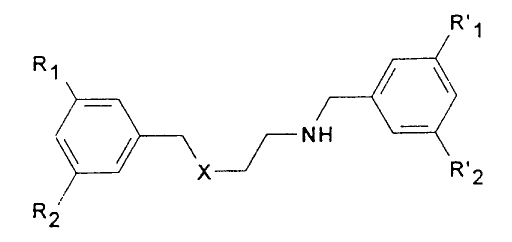
R₁ and R₂ are, independently of each other, H, -CH₃, -OCH₃, -CF₃, CN, -NO₂, a halogen atom
R'₁ and R'₂ are, independently of each other, -CH₃, -OCH₃, -CF₃, -CN, -NO₂, a halogen atom
X represents O or NH.

12. Use, in or for the preparation of a cosmetic, pharmaceutical or dermatological composition containing at least one product having an irritant side effect, of at least one compound of formula (I) below in order to reduce this irritant effect and/or to remove it altogether: 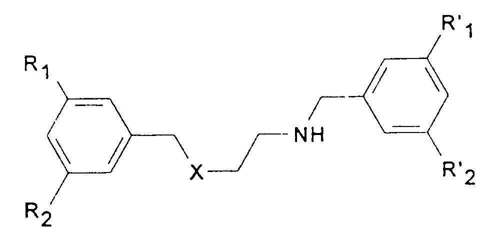
R₁ and R₂ are, independently of each other, H, -CH₃, -OCH₃, -CF₃, CN, -NO₂, a halogen atom
R'₁ and R'₂ are, independently of each other, -CH₃, -OCH₃, -CF₃, -CN, -NO₂, a halogen atom
X represents O or NH.

13. Use according to one of Claims 9 to 12, characterized in that X represents -NH.

14. Use according to any one of Claims 9 to 13, characterized in that the compound is used in an amount ranging from 0.000001 to 5% by weight relative to the total weight of the composition.

15. Use according to any one of Claims 9 to 14, characterized in that the compound is used in an amount ranging from 0.0001 to 0.1% by weight relative to the total weight of the composition.

16. Use according to any one of Claims 9 to 15, characterized in that the composition contains at least one agent chosen from antibacterial agents, antiparasitic agents, antifungal agents, antiviral agents, anti-inflammatory agents, antipruriginous agents, anaesthetics, keratolytic agents, anti-free-radical agents, antiseborrhoeic agents, antidandruff agents, antiacne agents and/or agents which modify cutaneous differentiation and/or proliferation and/or pigmentation.

17. Use according to any one of Claims 12 to 16, characterized in that the product having an irritant side effect is chosen from α-hydroxy acids, β-hydroxy acids, α-keto acids, β-keto acids, retinoids, anthralins, anthranoids, peroxides, minoxidil, lithium salts, anti-metabolites, vitamin D and derivatives thereof, hair dyes or toners, antiperspirants, depilatory agents or permanent-waving agents, perfumed alcoholic solutions, depigmenting agents, surfactants and solvents.

18. Cosmetic treatment process, characterized in that a composition containing, in a cosmetically acceptable medium, at least one compound of formula (I) below or one of the cosmetically, pharmaceutically or dermatologically acceptable salts thereof, is applied to the skin, to the hair and/or to the mucous membranes: 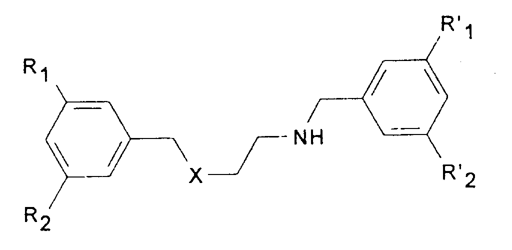
R₁ and R₂ are, independently of each other, H, -CH₃, -OCH₃, -CF₃, CN, -NO₂, a halogen atom
R'₁ and R'₂ are, independently of each other, -CH₃, -OCH₃, -CF₃, -CN, -NO₂, a halogen atom
X represents O or NH.

19. Process according to Claim 18, characterized in that X represents -NH.

20. Process according to Claim 18 or 19, characterized in that the compound is used in an amount ranging from 0.000001 to 5% by weight relative to the total weight of the composition.

21. Process according to one of Claims 18 to 20, characterized in that the compound is used in an amount ranging from 0.0001 to 0.1% by weight relative to the total weight of the composition.

## Patentansprüche

1. Kosmetische, pharmazeutische oder dermatologische Zusammensetzung, die in einem kosmetisch oder pharmazeutisch akzeptablen Medium mindestens ein Produkt mit reizender Nebenwirkung enthält,
**dadurch gekennzeichnet, daß**
sie ferner mindestens eine Verbindung der folgenden Formel (I) oder ein kosmetisch, pharmazeutisch oder dermatologisch akzeptables Salz dieser Verbindung enthält: 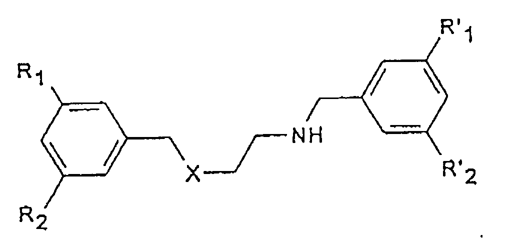 worin bedeuten:
R₁, R₂ unabhängig voneinander H, -CH₃, -OCH₃, -CF₃, -CN, -NO₂ oder ein Halogenatom,
R'₁, R'₂ unabhängig voneinander -CH₃, -OCH₃, -CF₃, -CN, -NO₂ oder ein Halogenatom,
X O oder NH.

2. Zusammensetzung nach Anspruch 1, dadurch gekennzeichnet, daß X NH bedeutet.

3. Zusammensetzung nach Anspruch 1 oder 2, dadurch gekennzeichnet, daß die Verbindung der Formel (I) in einem Mengenanteil von 0,000001 bis 5 Gew.-%, bezogen auf das Gesamtgewicht der Zusammensetzung, verwendet wird.

4. Zusammensetzung nach einem der Ansprüche 1 bis 3, dadurch gekennzeichnet, daß die Verbindung der Formel (I) in einem Mengenanteil von 0,0001 bis 0,1 Gew.-%, bezogen auf das Gesamtgewicht der Zusammensetzung, verwendet wird.

5. Zusammensetzung nach einem der Ansprüche 1 bis 4, dadurch gekennzeichnet, daß das Produkt mit reizender Nebenwirkung ausgewählt ist unter den α-Hydroxysäuren, β-Hydroxysäuren, α-Ketosäuren, β-Ketosäuren, Retinoiden, Anthralinen, Anthranoiden, Peroxiden, Minoxidil, Lithiumsalzen, Antimetaboliten, Vitamin D und seinen Derivaten, Haarfärbemitteln oder Haartönungen, Antitranspirantien, Enthaarungsmitteln oder Mitteln für permanente Haarwellungen, alkoholischen parfümierende Lösungen, depigmentierende Mitteln, grenzflächenaktiven Stoffen und Lösungsmitteln.

6. Zusammensetzung nach einem der Ansprüche 1 bis 5, dadurch gekennzeichnet, daß das Produkt mit reizender Nebenwirkung ein kosmetischer, pharmazeutischer oder dermatologischer Wirkstoff ist.

7. Zusammensetzung nach einem der Ansprüche 1 bis 6, dadurch gekennzeichnet, daß sie ferner mindestens ein Mittel enthält, das unter den antibakteriellen Mitteln, Mitteln gegen Parasiten, Mitteln gegen Pilze, Mitteln gegen Viren, entzündungshemmenden Mitteln, Mitteln gegen Juckreiz, Anästhetika, Keratolytika, Mitteln gegen freie Radikale, Antiseborrhoeika, Mitteln gegen Schuppen, Mitteln gegen Akne und/oder Mitteln, die die Differenzierung und/oder Proliferation und/oder Pigmentierung der Haut modulieren, ausgewählt ist.

8. Zusammensetzung nach einem der vorhergehenden Ansprüche, dadurch gekennzeichnet, daß das kosmetisch oder dermatologisch akzeptable Medium eine wäßrige oder wäßrig-alkoholische Lösung, eine Wasser-in-Öl-Emulsion, eine Öl-in-Wasser-Emulsion, eine Mikroemulsion, ein wäßriges Gel, ein wasserfreies Gel, ein Serum oder eine Vesikeldispersion ist.

9. Verwendung mindestens einer Verbindung der folgenden Formel (I):] 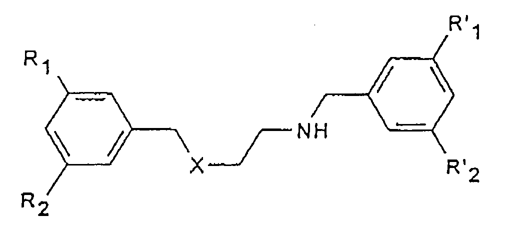 worin bedeuten:
R₁, R₂ unabhängig voneinander H, -CH₃, -OCH₃, -CF₃, -CN, -NO₂ oder ein Halogenatom,
R'₁, R'₂ unabhängig voneinander -CH₃, -OCH₃, -CF₃, -CN, -NO₂ oder ein Halogenatom,
X O oder NH
oder eines der kosmetisch, pharmazeutisch oder dermatologisch akzeptablen Salze dieser Verbindung als Substanz P-Antagonist in einer kosmetischen, pharmazeutischen oder dermatologischen Zusammensetzung zur Behandlung von mit empfindlicher Haut verbundenen Symptomen oder zur Herstellung dieser Verbindung.

10. Verwendung mindestens einer Verbindung der folgenden Formel (I): 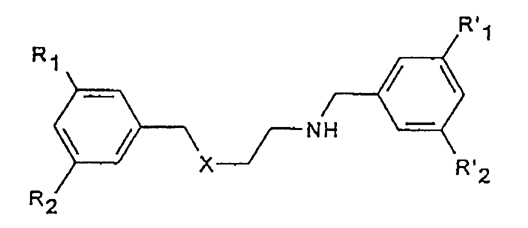 worin bedeuten:
R₁, R₂ unabhängig voneinander H, -CH₃, -OCH₃, -CF₃, -CN, -NO₂, oder ein Halogenatom,
R'₁, R'₂ unabhängig voneinander -CH₃, -OCH₃, -CF₃, -CN, -NO₂, oder ein Halogenatom,
X O oder NH
oder eines der kosmetisch, pharmazeutisch oder dermatologisch akzeptablen Salze dieser Verbindung als Lokalanalgetikum in einer kosmetischen, pharmazeutischen oder dermatologischen Zusammensetzung zur Behandlung empfindlicher Haut oder zur Herstellung dieser Zusammensetzung.

11. Verwendung mindestens einer Verbindung der folgenden Formel (I): 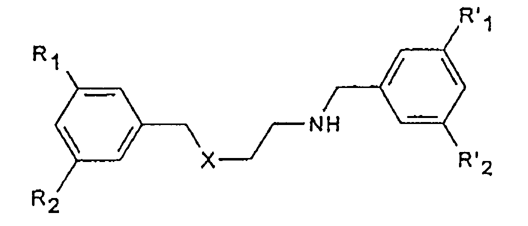 worin bedeuten:
R₁, R₂ unabhängig voneinander H, -CH₃, -OCH₃, -CF₃, -CN, -NO₂ oder ein Halogenatom,
R'₁, R'₂ unabhängig voneinander -CH₃, -OCH₃, -CF₃, -CN, -NO₂ oder ein Halogenatom,
X O oder NH
oder eines der kosmetisch, pharmazeutisch oder dermatologisch akzeptablen Salze dieser Verbindungen in einer kosmetischen, pharmazeutischen oder dermatologischen Zusammensetzung oder zu deren Herstellung zur Vorbeugung und/oder Bekämpfung von Hautreizungen und/oder Flechten und/oder Erythemen neurogenen Ursprungs und/oder schlechten Hautgefühls und/oder des Wärmegefühls und/oder des Pruritus der Haut und/oder des Pruritus der Schleimhäute.

12. Verwendung mindestens einer Verbindung der folgenden Formel (I): 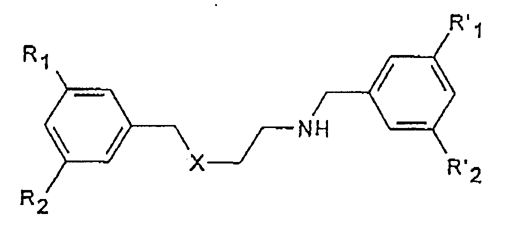 worin bedeuten:
R₁, R₂ unabhängig voneinander H, -CH₃, -OCH₃, -CF₃, -CN, -NO₂, oder ein Halogenatom,
R'₁, R'₂ unabhängig voneinander -CH₃, -OCH₃, -CF₃, -CN, -NO₂, oder ein Halogenatom,
X O oder NH
in einer kosmetischen, pharmazeutischen oder dermatologischen Zusammensetzung oder zu deren Herstellung, die mindestens ein Produkt mit reizender Nebenwirkung enthält, zur Verminderung und/oder Beseitigung dieser Reizwirkung.

13. Verwendung nach einem der Ansprüche 9 bis 12, dadurch gekennzeichnet, daß X -NH bedeutet.

14. Verwendung nach einem der Ansprüche 9 bis 13, dadurch gekennzeichnet, daß die Verbindung in einem Mengenanteil von 0,000001 bis 5 Gew.-%, bezogen auf das Gesamtgewicht der Zusammensetzung, verwendet wird.

15. Verwendung nach einem der Ansprüche 9 bis 14, dadurch gekennzeichnet, daß die Verbindung in einem Mengenanteil von 0,0001 bis 0,1 Gew.-%, bezogen auf das Gesamtgewicht der Zusammensetzung, verwendet wird.

16. Verwendung nach einem der Ansprüche 9 bis 15, dadurch gekennzeichnet, daß die Zusammensetzung mindestens ein Mittel enthält, das unter den antibakteriellen Mitteln, Mitteln gegen Parasiten, Mitteln gegen Pilze, Mitteln gegen Viren, entzündungshemmenden Mitteln, Mitteln gegen Juckreiz, Anästhetika, Keratolytika, Mitteln gegen freie Radikale, Antiseborrhoeika, Mitteln gegen Schuppen, Mitteln gegen Akne und/oder Mitteln, die die Differenzierung und/oder Proliferation und/oder Pigmentierung der Haut modulieren, ausgewählt ist.

17. Verwendung nach einem der Ansprüche 12 bis 16, dadurch gekennzeichnet, daß das Produkt mit reisender Nebenwirkung ausgewählt ist unter den α-Hydroxysäuren, β-Hydroxysäuren, α-Ketosäuren, β-Ketosäuren, Retinoiden, Anthralinen, Anthranoiden, Peroxiden, Minoxidil, Lithiumsalzen, Antimetaboliten, Vitamin D und seinen Derivaten, Haarfärbemitteln oder Haartönungen, Antitranspirantien, Enthaarungsmitteln oder Mitteln für permanente Haarwellungen, alkoholischen parfümierende Lösungen, depigmentierenden Mitteln, grenzflächenaktiven Stoffen und Lösungsmitteln.

18. Verfahren zur kosmetischen Behandlung, dadurch gekennzeichnet, daß auf die Haut, die Haare und/oder die Schleimhäute eine Zusammensetzung aufgetragen wird, die in einem kosmetisch akzeptablen Medium mindestens eine Verbindung der folgenden Formel (I): 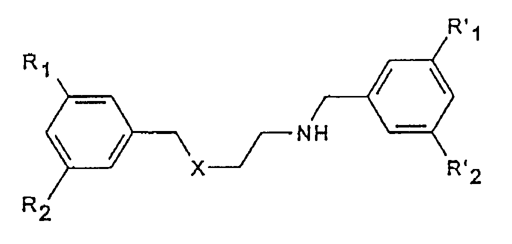 worin bedeuten:
R₁, R₂ unabhängig voneinander H, -CH₃, -OCH₃, -CF₃, -CN, -NO₂, oder ein Halogenatom,
R'₁, R'₂ unabhängig voneinander -CH₃, -OCH₃, -CF₃, -CN, -NO₂, oder ein Halogenatom,
X O oder NH
oder eines der kosmetisch, pharmazeutisch oder dermatologisch akzeptablen Salze dieser Verbindung enthält.

19. Verfahren nach Anspruch 18, dadurch gekennzeichnet, daß X -NH bedeutet.

20. Verfahren nach Anspruch 18 oder 19, dadurch gekennzeichnet, daß die Verbindung in einem Mengenanteil von 0,000001 bis 5 Gew.-%, bezogen auf das Gesamtgewicht der Zusammensetzung, verwendet wird.

21. Verfahren nach einem der Ansprüche 18 bis 20, dadurch gekennzeichnet, daß die Verbindung in einem Mengenanteil von 0,0001 bis 0,1 Gew.-%, bezogen auf das Gesamtgewicht der Zusammensetzung, verwendet wird.
